# EUROPÄISCHE PATENTSCHRIFT

(11) **EP 1 655 600 B1**
(45) Veröffentlichungstag und Bekanntmachung des Hinweises auf die Patenterteilung: **26.09.2012**
(21) Anmeldenummer: 05022480.7
(22) Anmeldetag: 14.10.2005
(51) Int. Cl.: G01N 21/89, G01N 33/36

(54) **Garnsensor**
Yarn sensor
Détecteur de fil

(30) Priorität: 06.11.2004 DE 102004053736
(43) Veröffentlichungstag der Anmeldung: 10.05.2006
(73) Patentinhaber: Oerlikon Textile GmbH & Co. KG, 42897 Remscheid (DE)
(72) Erfinder: Birlem, Olav, 41812 Erkelenz (DE)
(74) Vertreter: Hamann, Arndt

(56) Entgegenhaltungen:
- EP-A- 0 244 788
- EP-A- 0 754 943
- EP-A- 1 018 645
- EP-A- 1 035 059
- WO-A-93/13407
- WO-A-2005/068985

## Beschreibung

Die Erfindung betrifft einen Garnsensor zum optischen Abtasten eines in seiner Längsrichtung in einem Messspalt bewegten Garnes gemäß dem Oberbegriff des Anspruchs 1.

Zur berührungslosen Detektion von Garnparametern am laufenden Garn in einer Spinn- oder Spulmaschine werden häufig optische Systeme eingesetzt. Optische Systeme sind einfach in Aufbau und Funktion sowie kostengünstig ausführbar. Sie arbeiten mit Abschattung oder reflektiertem Licht. Dabei wird das Prüfgut von einer Lichtquelle beleuchtet.

Die EP 0 761 585 A1 beschreibt einen Garnsensor mit einem optisch arbeitenden System, der ebenfalls neben der Bestimmung des Garndurchmessers zur Erkennung von Fremdmaterial im Garn, wie Fremdfasern oder Verunreinigungen, dienen kann. Die Oberflächen im Messspalt, die von Licht, das von der Lichtquelle abgestrahlt wird, getroffen werden, reflektieren dieses auftreffende Licht. Das vom Garn reflektierte Licht stellt aufgrund der kleinen Oberfläche des Garns eine relativ geringe Signalquelle dar. Das in Strom umgewandelte Fadensignal bewegt sich im Nanoamperebereich. Die gegenüber der geringen bestrahlten Fläche des Garns relativ große, mit Schmutz behaftete Oberfläche des Messspaltes stellt wegen ihrer Ausdehnung eine nicht unerhebliche Reflexionssignalquelle dar. Die das Messergebnis verfälschende Störstrahlung wird auch als parasitäres Signal bezeichnet. Aufgrund der geringen Stärke des Fadensignals erfolgt eine hohe Verstärkung des aus der Lichteinstrahlung am Garn umgewandelten Signals , aber auch der parasitären Signale.

Dies führt zu einem unzulässig geringen Nutzsignal im Verhältnis zum Gesamtsignal. Der Garnsensor der EP 0 761 585 A1 vermag diesen Nachteil nicht zu beheben.

Die EP 0 244 788 A2 offenbart eine Vorrichtung zur Messung des Querschnitts eines laufenden Fadens mit einem Messspalt, an dessen einer Seite eine von einer Lichtquelle beleuchtete Streuscheibe angeordnet ist. Um eine möglichst homogene Beleuchtung im Messspalt zu erhalten, wird im Bereich der optischen Achse der Lichtquelle zwischen Streuscheibe und Messspalt eine das Licht abschattende Blende angeordnet. Dadurch wir die erhöhte Lichtintensität im Bereich der optische Achse reduziert.

Die CH 643 060 beschreibt wie die EP 0 761 585 A1 ebenfalls ein optisches System zur Prüfung des Garndurchmessers. Dabei wird ein dem Durchmesser des Garns proportionales Messsignal erzeugt. Signalschwankungen, die durch Änderungen der Lichtstärke der Lichtquelle infolge von Schwankungen der Speisespannung, Alterung oder Trübung auftreten, werden mit Hilfe schaltungstechnischer Mittel kompensiert. In einem dargestellten Ausführungsbeispiel wird von einer Punktlichtquelle ein Lichtkegel in Richtung auf einen Bildaufnehmer emittiert und das zwischen Lichtquelle und Bildaufnehmer durchlaufende Garn auf dem Bildaufnehmer als Schatten abgebildet. Als Bildaufnehmer dient ein CCD-Zeilensensor. Die Ausdehnung der Abschattung auf dem Bildaufnehmer ist abhängig vom Durchmesser des Garns. Die Lage des Garns, insbesondere der Abstand des Garns zum Bildaufnehmer, beeinflusst die Größe der abgeschatteten Fläche merklich. Bewegt sich beispielsweise das Garn bei gleich bleibendem Garndurchmesser auf den Bildaufnehmer zu, wird die Abschattung kleiner, obwohl der Garndurchmesser gleich geblieben ist. Dies führt zu Verfälschungen des Messergebnisses.

In einem weiteren in der CH 643 060 dargestellten alternativen Ausführungsbeispiel befindet sich zwischen der als punktförmig bezeichneten Lichtquelle und dem Garn eine Optik, die von der Lichtquelle emittiertes Licht als angenähert paralleles Strahlenbündel auf den Bildaufnehmer werfen soll. Auf diese Weise soll erreicht werden, dass die Abschattung und damit das Messergebnis nicht mehr von der Lage des Garns im Messbereich beziehungsweise im Messspalt beeinflusst wird. Bewegungen des laufenden Garns quer zur Laufrichtung im Messbereich sind in diesem Fall tolerierbar. Die Parallelität des Strahlenbündels ist davon abhängig, ob die Lichtquelle ideal punktförmig ist. Ideal punktförmige Lichtquellen stehen jedoch nicht zur Verfügung. Auch bei den als Punktlichtquellen bezeichneten Glühbirnen, die die CH 643 060 beschreibt, wird das Licht nicht in einem Punkt erzeugt. Das Licht wird üblicherweise in Glühbirnen mittels eines gehalterten Glühfadens erzeugt. Da die Voraussetzung einer punktförmigen Lichtquelle nicht erfüllbar ist, lassen sich auch die gleichmäßig verteilte Beleuchtungsstärke und die Parallelität des auf den Bildaufnehmer gerichteten Strahlenbündels nicht erreichen und bleiben mangelhaft. Ein von der Lage des Garns im Messspalt unabhängiges Messergebnis ist mit der Vorrichtung der CH 643 060 nicht erreichbar.

Eine andere Möglichkeit, gezielt paralleles Licht zu erzeugen, besteht in der Umwandlung des von einem Lambertschen Strahler emittierten Lichtes. Die Leuchtdichte eines Lambertschen Strahlers ist nach allen Richtungen des über der Licht emittierenden Fläche aufgespannten Halbraumes konstant. Der Lambertsche Strahler verhält sich also wie eine ideal diffus strahlende Fläche. Verbreitete derartige Oberflächenstrahler sind zum Beispiel Leuchtstofflampen, die jedoch wegen der erforderlichen Baugröße für den Einsatz in Garnmessköpfen an den Arbeitsstellen einer Spinn- oder Spulmaschine nicht geeignet sind. Leuchtdioden sind ebenfalls Flächenstrahler und werden in der Regel mit dem Attribut versehen, eine Lambertsche Abstrahlcharakteristik aufzuweisen. Laut Definition ist die von jedem Punkt der Fläche eines Lambertschen Strahlers emittierte Strahlung als divergentes Lichtbündel ausgebildet.

Die DE 23 37 413 B2 und die DE 198 59 274 A1 zeigen Vorrichtungen zur Überwachung eines laufenden Garns, bei denen als Lichtquellen Leuchtdioden und als Lichtempfänger Fotodioden eingesetzt werden.

Die gattungsbildende WO 93/13407 offenbart einen Garnsensor zum optischen Abtasten eines in seiner Längsrichtung in einem Messspalt bewegten Garnes, bei dem ein Lichtstrahl aus einer Lichtquelle in den Messspalt gesendet wird, mit mindestens einem Empfänger für direkt übertragenes Licht und Elementen zum Übertragen des Lichtes zwischen Lichtquelle, Messspalt und Empfängern. Die Lichtquelle ist vorzugsweise als Leuchtdiode ausgebildet.

Es ist Aufgabe der Erfindung, die Qualität der Messergebnisse eines Garnsensors zum optischen Abtasten eines in seiner Längsrichtung in einem Messspalt bewegten Garnes zu verbessern.

Diese Aufgabe wird mittels eines Garnsensors mit den Merkmalen des Anspruchs 1 gelöst.

Vorteilhafte Ausgestaltungen der Erfindung sind Gegenstand der Unteransprüche.

Die Erfindung beruht auf der Erkenntnis, dass die ausdrücklich als Lambertscher Strahler bezeichneten Lichtquellen für die Anwendung in einem Garnreiniger nur ungenügend an einen Lambertschen Strahler angenähertes Verhalten zeigen. Dies tritt aufgrund bautechnischer Toleranzen bei der Fertigung und Ungenauigkeiten der Positionierung bei der Montage auf. Diese mit Leuchtdioden arbeitenden Lichtquellen strahlen ein Lichtbündel ab, das nicht rotationssymmetrisch zur optischen Achse des aus Lichtquelle, Linse und Empfänger bestehenden Systems ausgebildet ist. Die asymmetrische Abstrahlung ("schielen") ändert sich von einer Leuchtdiode zur nächsten.

Durch die erfindungsgemäße Ausbildung des Garnsensors wird ermöglicht, dass sich das abgetastete Garn im Messspalt in einem Lichtbündel bewegt, das aus einer in seiner Beleuchtungsstärke sehr homogenen Strahlung und aus parallel zu einander und parallel zur optischen Achse verlaufenden Lichtstrahlen besteht. Damit wird bei einer Detektion des Garns mittels Abschattung eine gegenüber dem Stand der Technik wesentlich verbesserte Unabhängigkeit des Messergebnisses von der Lage des Garns im Messspalt erreicht. Durch Streulicht verursachter Lichtverlust, der die Messsignale schwächt, wird minimiert.

Vorteilhaft ist die Lichtquelle eine Leuchtdiode. Leuchtdioden benötigen nur wenig Bauraum und eignen sich daher besonders gut für den Einsatz an Arbeitsstellen von Spinn- oder Spulmaschinen, an denen nur sehr begrenzt Bauraum zur Verfügung steht.

Die Leuchtdiode ist vorzugsweise als Weißlichtleuchtdiode ausgebildet. Das Farbspektrum der Weißlichtleuchtdiode bietet bei der Farberkennung alle Möglichkeiten. Kostengünstig kann auf den Einsatz zusätzlicher Leuchtdioden mit Licht in anderen Farben verzichtet und der benötigte Bauraum gering gehalten werden. Da eine als alleinige Lichtquelle dienende Weißlichtleuchtdiode Licht in allen benötigten Farben emittiert, wird eine gleich bleibende Sensitivität des Garnsensors gegenüber unterschiedlichen Farben ermöglicht. Eine einzige Weißlichtleuchtdiode kommt dem Modell einer Punktlichtquelle wesentlich näher als eine Anordnung aus zwei oder mehreren Leuchtdioden.

Ein als Folie gemäß Anspruch 4 ausgebildeter Diffusor strahlt ein solches diffuses Licht ab, dass dieses eine geeignete Basis für die Bildung eines Lichtbündels mittels einer Linse entsprechend Anspruch 5 darstellt. Als Diffusor kann dabei eine Folie des Typs Oracal 8005, Serie Translucent, der Fa. K. Gröner verwendet werden. Diese Folie wurde bisher für Werbeaufkleber eingesetzt und damit auf einem Einsatzgebiet, das völlig andersartig ist als ein Einsatz in einem Garnsensor zur Erhöhung der Messgenauigkeit wie bei der vorliegenden Erfindung. Das Lichtbündel weist nach dem Passieren der Linse vorteilhaft eine homogen verteilte Beleuchtungsstärke und einen zur optischen Achse der Linse quasiparallelen Strahlengang auf, wobei das gesamte Strahlenbündel eine Divergenz aufweist, die gegen Null geht. Ein punktförmiger Fleck, zum Beispiel aufgrund einer Verunreinigung, führt nicht zur Inhomogenität des Lichtbündels im Messfeld, solange die restliche leuchtende Folie Licht abstrahlt, das den Anforderungen an die Lambertsche Abstrahlcharakteristik genügt.

Mit einem Garnsensor gemäß Anspruch 6 sowie Anspruch 7 ist eine verbesserte Detektion von Fremdstoffen im Garn erzielbar. Nur vom Garn reflektiertes Licht der Lichtquelle erreicht die zwei Empfänger für reflektiertes Licht. Störungen und Verfälschungen durch so genannte parasitäre Signale lassen sich vermeiden. Die Messempfindlichkeit des Garnsensors kann sensibler eingestellt werden.

Eine Blende nach Anspruch 8 trägt dazu bei, dass nur Licht der Lichtquelle in den Messspalt gelangt, das homogen verteilt und parallel zur optischen Achse der vor der Lichtquelle angeordneten Linse ausgerichtet ist. Eine Blende nach Anspruch 9 begrenzt das auf das Garn gerichtete Lichtbündel derart, dass nur Flächen beleuchtet werden, die keine Reflexe verursachen, die zu parasitären Signalen führen.

Mit einem erfindungsgemäßen Garnsensor wird die Qualität des Messergebnisses bei der Bestimmung des Garndurchmessers und bei der Fremdfasererkennung verbessert. Mittels einer Lichtquelle und der optischen Transformation gemäß der Erfindung wird eine bessere Homogenität des Lichtbündels erzielt. Daher ist ein breiteres Messfeld möglich bei gleichzeitiger Verbesserung der Homogenität im Messfeld. Das führt dazu, dass eine größere Fadenbewegung im Messfeld toleriert und gegebenenfalls auf Fadenführer zur Führung des Garns im Messspalt verzichtet werden kann. Auf diese Weise lässt sich baulicher Aufwand einsparen und der Faden wird weniger geschädigt. Die Verschmutzung im Bereich des Garnsensors durch Abrieb wird gesenkt.

Weitere Einzelheiten der Erfindung sind den Darstellungen der Figuren entnehmbar.

### Es zeigt:

- Fig. 1: eine Prinzipdarstellung einer Spinnstelle,
- Fig. 2: einen Garnsensor mit geöffnetem Gehäuse,
- Fig. 3: eine Anordnung von Elementen des Garnsensors der Fig. 2,
- Fig. 4: eine Prinzipdarstellung eines Lambertschen Flächenstrahlers.

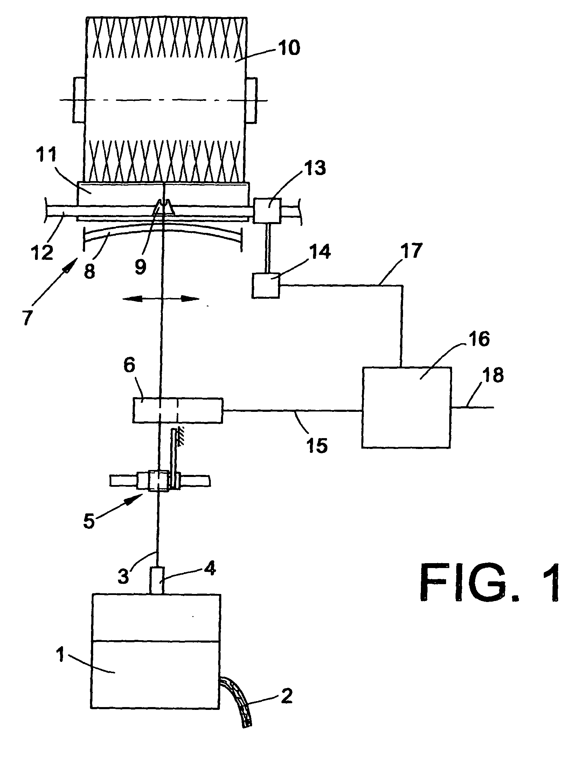

Fig. 1 zeigt eine Spinnbox 1 einer Open-End-Spinnmaschine, der ein Faserband 2 zugeführt wird. Das in der Spinnbox 1 erzeugte Garn 3 wird über das Abzugsröhrchen 4 mittels des Abzugswalzenpaares 5 abgezogen, durchläuft einen Garnsensor 6 und wird über einen Bügel 8 durch die Hin- und Herbewegung des Fadenführers 9 einer Changiereinrichtung 7 über eine vorgegebene Breite zu einer Kreuzspule 10 aufgewickelt. Die Kreuzspule 10 wird mittels einer Friktionswalze 11 angetrieben. Der Fadenführer 9 ist auf einer Fadenführerstange 12 befestigt, die von einem Fadenführergetriebe 13 hin- und herbewegt wird. Der Antrieb des Fadenführergetriebes 13 erfolgt durch eine Antriebseinrichtung 14. Der Garnsensor 6 zur Überwachung des laufenden Garns 3 ist oberhalb des Abzugswalzenpaares 5 im Changierbereich des Garns 3 angeordnet. In einer nicht dargestellten alternativen Ausführungsform kann der Garnsensor 6 dem Abzugswalzenpaar 5 vorgeordnet statt nachgeordnet sein. Der Garnsensor 6 ist über eine Leitung 15 mit der Steuerungseinrichtung 16 verbunden, die die vom Garnsensor 6 emittierten Signale empfängt. Über eine weitere Leitung 17 ist die Steuerungseinrichtung 16 mit der Antriebseinrichtung 14 verbunden. Die Antriebseinrichtung 14 ist als Elektromotor ausgeführt. Über die Leitung 18 ist die Steuerungseinrichtung 16 mit weiteren hier nicht dargestellten Spinnstellen, Datenverarbeitungseinrichtungen oder Spinnmaschinen verbunden.

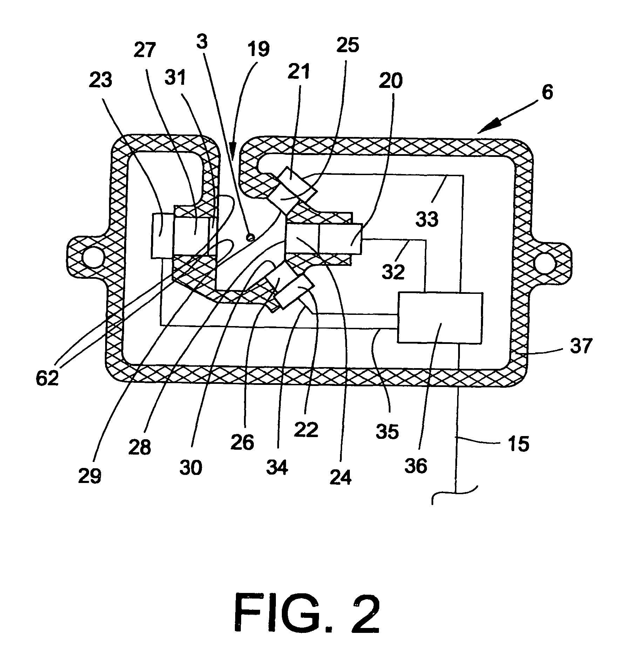

Der Fig. 2 ist die Lage einzelner Bestandteile des Garnsensors 6 zum Messspalt 19 und zum Garn 3 entnehmbar. Die als Leuchtdiode 20 ausgebildete Lichtquelle sowie die Fotodioden 21, 22, die dem Empfang vom Garn 3 reflektierten Lichtes dienen, sind in der Darstellung der Fig. 2 rechts vom Messspalt 19 positioniert. Die Fotodiode 23 zum Empfang des direkt von der Leuchtdiode 20 übertragenen Lichts ist in der Darstellung der Fig. 2 links vom Messspalt 19 positioniert. Zwischen der Leuchtdiode 20 und dem Messspalt 19 einerseits sowie zwischen dem Messspalt 19 und den Fotodioden 21, 22, 23 andererseits sind Elemente 24, 25, 26, 27 zum Übertragen des Lichts angeordnet. Die Elemente 24, 25, 26, 27 zum Übertragen des Lichts sind durch Fenster 28, 29, 30, 31 vom Messspalt 19 getrennt. Die Fenster können einen Schutz der Elemente 24, 25, 26, 27 zum Übertragen des Lichts vor Verschmutzung durch Staub und Faserflug bewirken. Die Leuchtdiode 20 und die Fotodioden 21, 22, 23 sind jeweils mittels der Leitungen 32, 33, 34, 35 mit einer Signalverarbeitungseinrichtung 36 verbunden. Die Signalverarbeitungseinrichtung 36 ihrerseits ist über die Leitung 15, die durch das Gehäuse 37 des Garnsensors 6 nach außen führt, mit der Steuerungseinrichtung 16 verbunden.

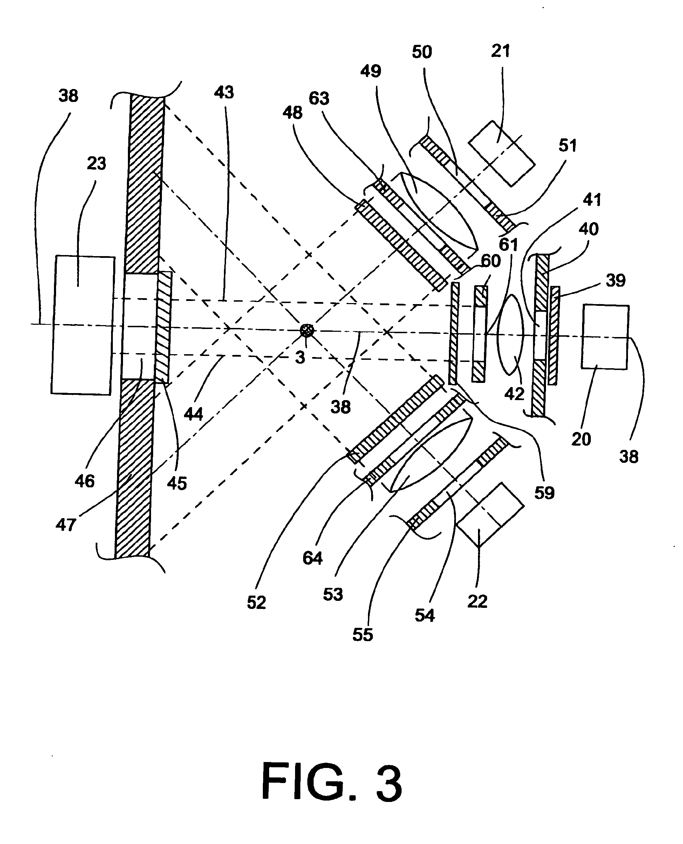

Fig. 3 zeigt eine Anordnung von Bauteilen des Garnsensors 6, der zur Erkennung von Fremdstoffen im Garn 3 geeignet ist. Als Lichtquelle dient die Leuchtdiode 20, die annähernd die Abstrahlcharakteristik eines Lambertschen Strahlers aufweist. Die Leuchtdiode 20 ist als Weißlichtleuchtdiode ausgebildet. Weißlichtleuchtdioden emittieren Licht mit breitem Emissionsspektrum. Auf den Einsatz von mehreren Leuchtdioden zur Emission unterschiedlicher Farben oder zur Verstärkung des emittierten Lichts kann bei Verwendung einer Weißlichtleuchtdiode verzichtet werden. Für Leuchtdioden ist die Kurzbezeichnung "LED" gebräuchlich. Das von der Leuchtdiode 20 emittierte Licht passiert das Element 24 zum Übertragen des Lichts. Das Element 24 umfasst eine Folie 39, eine Blende 40 mit der Blendenöffnung 41, eine Linse 42, eine Blende 60 mit rechteckiger Blendenöffnung 61 und eine Glasscheibe 59, die nacheinander vom Licht in Richtung der optischen Achse 38 durchquert werden. Die Blende 40 weist eine Blendenöffnung 41 mit einer Weite von 1 mm auf. Die Folie 39 strahlt divergente Lichtbündel ab und weist die Abstrahlcharakteristik eines Lambertschen Strahlers auf. Als Folie 39 kommt beispielsweise der Folientyp Oracal 8500, Serie Translucent, der Fa. K. Gröner zum Einsatz. Nach der Linse 42 sind die einzelnen Lichtstrahlen quasiparallel zueinander in Richtung der optischen Achse 38 ausgerichtet und homogen über den Querschnitt des Gesamtlichtstrahls verteilt. Der Gesamtlichtstrahl ist durch die beiden gestrichelt dargestellten Linien 43, 44 repräsentiert. Die Folie 39 bildet eine virtuelle Lichtquelle, die im Unendlichen abgebildet wird. Auf dem Weg des Lichtbündels zwischen Linse 42 und der Bildebene der Fotodiode 23 ist immer das Abbild der virtuellen Lichtquelle vorhanden. Das Abbild ist jedoch unscharf. Dieser Effekt geht mit einer weiteren Homogenisierung des Lichtbündels einher. Das Garn 3 quert den Verlauf des Gesamtlichtstrahls und wird in Form einer Abschattung auf der Fotodiode 23 abgebildet. Zwischen dem Garn 3 und der Fotodiode 23 passiert der Gesamtlichtstrahl die Glasscheibe 45 sowie die Blendenöffnung 46 der Blende 47. Vom Garn 3 wird von der Leuchtdiode 20 emittiertes Licht reflektiert. Die Fotodioden 21, 22 erfassen einen Teil des reflektierten Lichtes. Zwischen Garn 3 und den Fotodioden 21, 22 passiert das reflektierte Licht jeweils die Elemente 25, 26. Die Elemente 25, 26 umfassen jeweils die zugehörige Glasscheibe 48, 52, die Blende 63,64, die Linse 49, 53 und die Blendenöffnung 50, 54 der Blende 51, 55. Die Elemente 25, 26 zur Übertragung des reflektierten Lichts sind so ausgebildet und angeordnet, dass durch die Fotodioden 21, 22 in Abwesenheit des Garns 3 jeweils Abbildungen der gegenüberliegenden Oberflächen, zum Beispiel der Blende 40 oder der Wand 62 des Messspaltes 19, erfassbar sind. Diese Oberflächen liegen beiderseitig außerhalb der von der direkten Strahlung der Leuchtdiode 20 beleuchteten Fläche der Wand 62 des Messspaltes 19.

Alternativ können die Blenden 51 und 55 auch entfallen. Die Glasscheiben 48, 52, 59 können in einer weiteren alternativen Ausführung als Blenden ausgebildet sein und rechteckige Blendenöffnungen aufweisen.

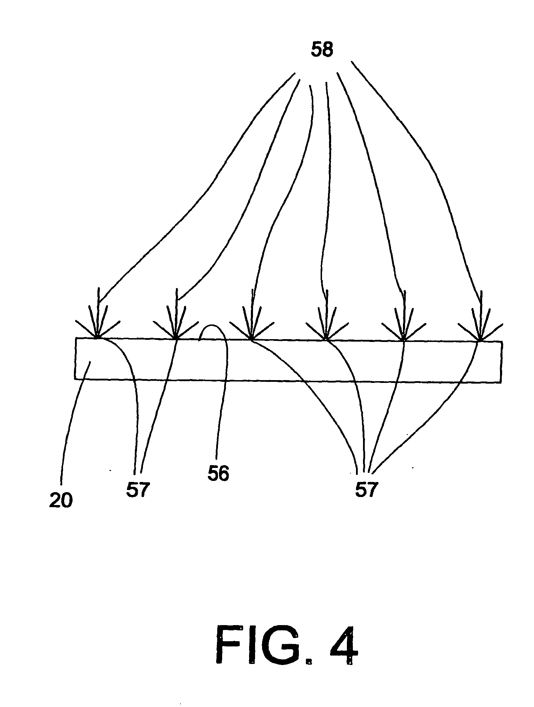

Fig. 4 zeigt die Prinzipdarstellung der Leuchtdiode 20, deren Licht abstrahlende Fläche 56 die Charakteristik eines Lambertschen Strahlers aufweist. Von jedem Punkt 57 der Fläche 56 wird ein divergentes Strahlenbündel 58 emittiert. Das Licht, das ein Lambertscher Strahler emittiert, lässt sich in ein homogenes Licht mit quasiparallelem Strahlengang umwandeln, wobei die Homogenität und die Parallelität des Lichtes besser sind, als es der Fall ist, wenn übliche so genannte Punktlichtquellen eingesetzt werden.

## Patentansprüche

1. Gransensor (6) zum optischen Abtasten eines in seiner Längsrichtung in einem Messspalt (19) bewegten Garnes (3), bei dem ein Lichtstrahl aus einer Lichtquelle (20) in den Messspalt gesendet wird, mit mindestens einem Empfänger (23) für direkt übertragenes Licht und Elementen (24, 25, 26, 27) zum Übertragen des Lichts zwischen Lichtquelle, Messspalt und Empfängern,
wobei die Lichtquelle (20) ein Strahler ist, der in seiner Abstrahlcharakteristik einem Lambertschen Strahler nahe kommt,
**dadurch gekennzeichnet, dass** das Licht übertragende Element (24), welches zwischen Lichtquelle (20) und Messspalt (19) angeordnet ist, in Strahlungsrichtung hinter der Lichtquelle (20) eine Blende (40) mit einer Blendenöffnung (41) und eine Linse (42) enthält, die so ausgebildet und angeordnet sind, dass die Blende (40) zumindest annähernd im Unendlichen abgebildet wird und dass zwischen der Lichtquelle (20) und dieser vorgeschalteten Blende (40) ein Diffusor (39) angeordnet und derart ausgebildet ist, dass er die die Blendenöffnung (41) der Blende (40) passierende Strahlung symmetrisch zur optischen Achse (38) der Linse (42) formt.

2. Garnsensor nach Anspruch 1, **dadurch gekennzeichnet, dass** die Lichtquelle (20) eine Leuchtdiode ist.

3. Garnsensor nach Anspruch 2, **dadurch gekennzeichnet, dass** die Leuchtdiode (20) eine Weißlichtleuchtdiode ist.

4. Garnsensor nach einem der Ansprüche 1 bis 3, **dadurch gekennzeichnet, dass** der Diffusor (39) eine Folie ist, die die ankommende sie durchdringende Strahlung als divergente Lichtbündel abstrahlt, wobei das abgestrahlte Licht die Charakteristik eines von einem Lambertschen Strahler emittierten Lichtes aufweist.

5. Garnsensor nach einem der Ansprüche 1 bis 5, **dadurch gekennzeichnet, dass** die Eintrittsfläche der vor der Lichtquelle (20) angeordneten Linse (42) so ausgebildet ist, dass die Strahlung in Richtung der optischen Achse (38) der Linse (42) eine homogene Verteilung der Beleuchtungsstärke ausweist und die Austrittsfläche der Linse (42) so ausgebildet ist, dass die von der Eintrittsfläche der Linse (42) kommende Strahlung nahezu parallelisiert und quasiparallel zur optischen Achse (38) der Linse (42) abgestrahlt wird.

6. Garnsensor nach einem der Ansprüche 1 bis 5, **dadurch gekennzeichnet, dass** der Garnsensor (6) neben einem Empfänger (23) für direkt übertragenes Licht zwei weitere Empfänger (21, 22) für vom Garn (3) reflektiertes Licht zum Detektieren von Fremdfasern umfasst.

7. Garnsensor nach Anspruch 6, **dadurch gekennzeichnet, dass** die das Licht übertragenden Elemente (25, 26), welche zwischen Messspalt (19) und Empfängern (21, 22) für reflektiertes Licht angeordnet sind, in Strahlungsrichtung des reflektierten Lichts vor den das reflektierende Licht erfassenden Empfänger (21, 22) jeweils eine so ausgebildete und angeordnete weitere Linse (49, 53) enthalten, dass durch die Empfänger (21, 22) in Abwesenheit des Garnes (3) jeweils Abbildungen auf der gegenüberliegenden Wand (62) des Messspaltes (19) erfassbar sind, die im Wesentlichen beiderseitig außerhalb der Abbildung der Lichtquelle (20) auf der gegenüberliegenden Wand (62) des Messspaltes (19) liegen.

8. Garnsensor nach einem der vorhergehenden Ansprüche, **dadurch gekennzeichnet, dass** die Blende (40) zwischen Lichtquelle (20) und Linse (42) eine Blendenöffnung (41) aufweist, deren Weite 0,8 mm bis 1,2 mm, vorzugsweise 1 mm, beträgt.

9. Garnsensor nach einem der vorhergehenden Ansprüche, **dadurch gekennzeichnet, dass** zwischen der der Folie (39) im Strahlengang nachfolgenden Linse (42) und dem Garn (3) eine Blende (60) mit rechteckiger Blendenöffnung (61) angeordnet ist.

## Claims

1. Yarn sensor (6) for optically scanning a yarn (3) travelling in its longitudinal direction in a measuring gap (19), in which a light beam is transmitted from a light source (20) into the measuring gap, comprising at least one receiver (23) for directly transmitted light and elements (24, 25, 26, 27) for transmitting the light between the light source, measuring gap and receivers, wherein the light source (20) is an emitter which is close to a Lambert emitter in its emission characteristic,
**characterised in that** the element (24) transmitting light, which is arranged between the light source (20) and the measuring gap (19), comprises in radiation direction downstream of the light source (20) a screen (40) with a screen opening (41) and a lens (42), which are configured and arranged so that the screen (40) is projected at least approximately into infinity, and **in that** between the light source (20) and said screen (40) arranged upstream thereof a diffuser (39) is arranged and is configured such that it shapes the radiation passing through the screen opening (41) of the screen (40) symmetrically to the optical axis (38) of the lens (42).

2. Yarn sensor according to claim 1, **characterised in that** the light source (20) is a light-emitting diode.

3. Yarn sensor according to claim 2, **characterised in that** the light-emitting diode (20) is a white light LED.

4. Yarn sensor according to any one of claims 1 to 3, **characterised in that** the diffuser (39) is a film for radiating the incoming radiation penetrating therethrough as divergent light bundles, wherein the radiated light has the characteristic of light emitted by a Lambert emitter.

5. Yarn sensor according to any one of claims 1 to 5, **characterised in that** the admission surface of the lens (42) arranged upstream of the light source (20) is configured so that the radiation has a homogenous distribution of light intensity in the direction of the optical axis (38) of the lens (42) and the discharge surface of the lens (42) is configured so that the radiation coming from the admission surface of the lens (42) is virtually parallelised and is radiated virtually parallel to the optical axis (38) of the lens (42).

6. Yarn sensor according to any one of claims 1 to 5, **characterised in that** the yarn sensor (6) comprises in addition to a receiver (23) for directly transmitted light two additional receivers (21, 22) for light reflected by the yarn (3) for detecting foreign fibres.

7. Yarn sensor according to claim 6, **characterised in that** the elements (25, 26) transmitting light, which are arranged between the measuring gap (19) and receivers (21, 22) for reflected light, comprise in radiation direction of the reflected light upstream of the receivers (21, 22) detecting the reflected light a thus configured and arranged additional lens (49, 53), **in that** by means of the receivers (21, 22) in the absence of the yarn (3) projected images can be detected on the opposite wall (62) of the measuring gap (19), which are essentially on both sides outside the projected image of the light source (20) on the opposite wall (62) of the measuring gap (19).

8. Yarn according to any one of the preceding claims, **characterised in that** the screen (40) comprises a screen opening (41) between the light source (20) and lens (42), the width of which is 0.8 mm to 1.2 mm, preferably 1 mm.

9. Yarn sensor according to any one of the preceding claims, **characterised in that** between the lens (42) following the film (39) in the beam path and the yarn (3) a screen (60) with a rectangular screen opening (61) is arranged.

## Revendications

1. Détecteur de fil (6) pour l'analyse optique d'un fil (3) déplacé dans sa direction longitudinale dans une fente de mesure (19), dans lequel un faisceau lumineux est envoyé dans la fente de mesure depuis une source de lumière (20), avec au moins un récepteur (23) pour la lumière transmise directement et des éléments (24, 25, 26, 27) pour transmettre la lumière entre source de lumière, fente de mesure et récepteurs, la source de lumière (20) étant un émetteur dont la caractéristique de rayonnement se rapproche de celle d'un émetteur de Lambert,
**caractérisé en ce que** l'élément transmetteur de lumière (24) qui est disposé entre source de lumière (20) et fente de mesure (19) contient, derrière la source de lumière (20) dans la direction de rayonnement, un diaphragme (40) avec une ouverture de diaphragme (41) et une lentille (42) qui sont conçus et disposés de telle sorte que le diaphragme (40) soit reproduit au moins approximativement à l'infini et qu'un diffuseur (39) est disposé entre la source de lumière (20) et le diaphragme (40) placé devant celle-ci et conçu de sorte à donner au rayonnement qui passe par l'ouverture de diaphragme (41) du diaphragme (40) une forme symétrique par rapport à l'axe optique (38) de la lentille (42).

2. Détecteur de fil selon la revendication 1, **caractérisé en ce que** la source de lumière (20) est une diode électroluminescente.

3. Détecteur de fil selon la revendication 2, **caractérisé en ce que** la diode électroluminescente (20) émet une lumière blanche.

4. Détecteur de fil selon une des revendications 1 à 3, **caractérisé en ce que** le diffuseur (39) est un film qui émet le rayonnement arrivant qui le traverse sous la forme de faisceaux lumineux divergents, la lumière émise présentant la caractéristique d'une lumière émise par un émetteur de Lambert.

5. Détecteur de fil selon une des revendications 1 à 5, **caractérisé en ce que** la surface d'entrée de la lentille (42) disposée devant la source de lumière (20) est conçue de telle sorte que le rayonnement en direction de l'axe optique (38) de la lentille (42) présente une répartition homogène de l'intensité d'éclairement et la surface de sortie de la lentille (42) est conçue de telle sorte que le rayonnement venant de la surface d'entrée de la lentille (42) soit rendu quasiment parallèle et émis quasi parallèlement à l'axe optique (38) de la lentille (42).

6. Détecteur de fil selon une des revendications 1 à 5, **caractérisé en ce que** le détecteur de fil (6) comprend, en plus d'un récepteur (23) pour la lumière transmise directement, deux autres récepteurs (21, 22) pour la lumière réfléchie par le fil (3) pour détecter des fibres étrangères.

7. Détecteur de fil selon la revendication 6, **caractérisé en ce que** les éléments transmetteurs de lumière (25, 26) qui sont disposés entre la fente de mesure (19) et les récepteurs (21, 22) pour la lumière réfléchie contiennent, devant les récepteurs (21, 22) détectant la lumière réfléchie dans la direction de rayonnement de la lumière réfléchie, chacun une autre lentille (49, 53) conçue et disposée de telle sorte que les récepteurs (21, 22) permettent, en l'absence du fil (3), de détecter des reproductions sur la paroi opposée (62) de la fente de mesure (19) qui se situent sensiblement de chaque côté en dehors de la reproduction de la source de lumière (20) sur la paroi opposée (62) de la fente de mesure (19).

8. Détecteur de fil selon une des revendications précédentes, **caractérisé en ce que** le diaphragme (40) entre source de lumière (20) et lentille (42) présente une ouverture de diaphragme (41) dont la largeur est comprise entre 0,8 mm et 1,2 mm, de préférence égale à 1 mm.

9. Détecteur de fil selon une des revendications précédentes, **caractérisé en ce qu'**un diaphragme (60) avec ouverture de diaphragme rectangulaire (61) est disposé entre la lentille (42) qui suit le film (39) dans le chemin optique et le fil (3).
